# EUROPEAN PATENT APPLICATION

(11) **EP 1 818 061 A1**
(43) Date of publication of application: **15.08.2007**
(21) Application number: 05026371.4
(22) Date of filing: 02.12.2005
(51) Int. Cl.: A61K 38/27, A61P 17/14

(54) **Use of ghrelin for stimulating hair growth**

(71) Applicant: Charité-Universitätsmedizin Berlin, 10117 Berlin (DE)
(72) Inventor: Springer, Jochen, Falkensee, 14612 (DE); Anker, Stefan, Berlin, 13088 (DE); Palus, Sandra, Berlin, 10559 (DE)
(74) Representative: Krauss, Jan

(57) **Abstract**

The present invention relates to ghrelin, an analogue thereof and a pharmaceutically acceptable salt thereof in the preparation of a medicament for administering to a mammal so as to stimulate its hair growth.

SEQUENCE LISTING

<110>  Universitätsmedizin Charitè

<120>  Use of ghrelin, an analogue thereof or a pharmaceutically
       acceptable salt thereof

<130>  FB16234

<160>  5

<170>  PatentIn version 3.2

<210>  1
<211>  28
<212>  PRT
<213>  Homo sapiens

<400>  1

Gly Ser Ser Phe Leu Ser Pro Glu His Gln Arg Val Gln Gln Arg Lys
1               5                   10                  15

Glu Ser Lys Lys Pro Pro Ala Lys Leu Gln Pro Arg
            20                  25

<210>  2
<211>  27
<212>  PRT
<213>  Rattus norvegicus

<400>  2

Gly Ser Ser Phe Leu Ser Pro Glu His Gln Lys Ala Gln Arg Lys Glu
1               5                   10                  15

Ser Lys Lys Pro Pro Ala Lys Leu Gln Pro Arg
            20                  25

<210>  3
<211>  28
<212>  PRT
<213>  Mus musculus

<400>  3

Gly Ser Ser Phe Leu Ser Pro Glu His Gln Lys Ala Gln Gln Arg Lys
1               5                   10                  15

Glu Ser Lys Lys Pro Pro Ala Lys Leu Gln Pro Arg
            20                  25

<210>  4
<211>  28
<212>  PRT
<213>  Artificial

<220>
<223>  Ghrelin analogue

<400>  4

Gly Ser Ser Phe Leu Ser Pro Glu His Gln Lys Leu Gln Gln Arg Lys
1               5                   10                  15

Glu Ser Lys Lys Pro Pro Ala Lys Leu Gln Pro Arg
            20                  25

<210>  5
<211>  28
<212>  PRT
<213>  Artificial

<220>
<223>  Ghrelin analogue

<400> 5

Gly Ser Ser Phe Leu Ser Pro Glu His Gln Lys Val Gln Gln Arg Lys
1               5                   10                  15

Glu Ser Lys Lys Pro Pro Ala Lys Leu Gln Pro Arg
            20                  25

## Description

This invention relates to ghrelin, an analogue thereof and a pharmaceutically acceptable salt thereof in the preparation of a medicament for administering to a mammal so as to stimulate its hair growth.

Ghrelin is a recently discovered gastric hormone of 28 amino acids showing a unique structure with an n-octanoyl ester at its third serine residue (Kojima M et al. Nature 1999; 402(6762):656660). Though many synthetic peptidyl and nonpeptidyl growth hormone (GH) secretagogues (GHS) were identified as ligands of GHS-R, ghrelin is shown to be a physiological ligand for the GHS-R. Ghrelin powerfully stimulates GH secretion through its interaction with GHS-R both in animals and in humans (Ukkola, O et al., 2002 Ann. Med. 34:102-108). The GH-releasing activity of ghrelin is mediated by activation of GHS-R at the pituitary and, mainly, at the hypothalamic level (Kojima M et al. Nature 1999; 402(6762):656660) likely by enhancing the activity of growth hormone releasing hormone (GHRH)-secreting neurons and, concomitantly, acting as a functional somatostatin (SS) antagonist (Ghigo E et al. Eur J Endocrinol 1997; 136(5):445460). Other mechanisms have been postulated recently as well (Ahnfelt-Ronne I et al. Endocrine 2001; 14(1):133-135).

The GHS-R and its subtypes are not restricted to the hypothalamus-pituitary unit but are present also in other central and peripheral tissues (Papotti M et al. J Clin Endocrinol Metab 2000; 85(10):3803-3807) and the physiological actions of ghrelin, as well as those of synthetic GHS are not restricted to GH secretion. In fact, ghrelin stimulates lactotroph and corticotroph hormone secretion, has orexigenic and cardiovascular actions, shows anti proliferative effects on thyroid and breast tumors and regulates gastric motility and acid secretion through vagal mediation (Ukkola, O et al., 2002, Anti. Med. 34:102-108).
In humans, fasting leads to elevated serum GH concentrations. Traditionally, changes in hypothalamic GHRH and somatostatin have been considered as the main mechanisms, which induce elevations in GH secretion during fasting. As ghrelin administration in man also stimulates GH release, and serum ghrelin concentrations are elevated during fasting, increased ghrelin actions might be another mechanism whereby fasting results in the stimulation of GH release.

Although ghrelin is likely to regulate pituitary GH secretion in interplay with GHRH and SS; GHS receptors have also been identified on hypothalamic neurons and in the brainstem (Nakazato M et al. Nature 2001; 409(6817):194-198). Apart from potential paracrine effects, ghrelin may thus offer an endocrine link between the stomach, hypothalamus and pituitary, suggesting an involvement in the regulation of energy balance. Tschop et al. have shown that daily peripheral administration of ghrelin in mice and rats caused weight gain by reducing fat utilization (Tschop M et al. Nature 2000; 19; 407(6806):908-913). Intracere broventricular administration of ghrelin generated a dosedependent increase in food intake and body weight. Rat serum ghrelin concentrations increased by fasting and decreased by re-feeding or oral glucose administration, but not by water ingestion. Apparently ghrelin, in addition to its role in regulating GH secretion, signals the hypothalamus when an increase in metabolic efficiency is necessary (Tschop M et al. Nature 2000; 19; 407(6806):908-913; Muller A F et al.. Clin Endocrnol (Oxf) 2001; 55(4):461-467).

Studies by Kojima and others have shown that unacylated ghrelin (UAG) has no affinity to the known GHS-R ((GHS-Rla receptor), which is responsible for GH release from the pituitary gland (Kojima M et al. Nature 1999; 402(6762):656-660). This was confirmed later by Bednarek M A et al (Bednarek M A et al, J. Med Chem. 2000, 43:4370-4376), who showed that unacylated ghrelin could not be a physiological ligand of the GHS-Rla receptor (IC50>10,000 nM), since it poorly activated GHS-Rla at micromolar concentrations; large hydrophobic acyl group is obligatory at position 3 of ghrelin for its biological response on GH secretion.

Ghrelin was discovered as the peptide hormone that stimulates release of growth hormone from the anterior pituitary. It was subsequently determined that ghrelin, along with several other hormones, has significant effects on appetite and energy balance.
Further, ghrelin analogues have been proposed for glycemic control in certain metabolic diseases (US 2005/0080007).
Subject of the present invention is the surprising effect of ghrelin, analogues thereof and pharmaceutically acceptable salts thereof in the preparation of a medicament for administering to a mammal so as to stimulate its hair growth. Especially preferred is human ghrelin.
therefore, subject of the present invention is the use of an agent selected from the group comprising ghrelin, an analogue thereof and a pharmaceutically acceptable salt thereof in the preparation of a medicament for administering to a mammal so as to stimulate its hair growth.
Ghrelin is a amino acid peptide of SEQ ID No. 1. Analogue of ghrelin according to this invention means
a) Peptide-analogs, full length or short peptides that mimic ghrelin action on the GHS-receptor and other putative pathways.
b) Non-peptide-analogs that mimic ghrelin action on the GHS-receptor and other putative pathways.

Unless otherwise stated, those amino acids with a chiral center are provided in the L-enantiomer.

The present invention also encompasses derivatives of the inventive amino acid peptides. Reference to "a derivative thereof" refers to a modified amino acid such as the corresponding D-amino acid, a N-alkyl-amino acid, a labeled amino acid.

Preferred derivatives of analogs of the invention comprise D-amino adds, N-alkyl-amino acids, β-amino acids, and/or one or more labeled amino adds (including a labeled version of a D-amino acid, a N-alkyl-amino acids, or a β-amino add). A labeled derivative indicates the alteration of an amino acid or amino add derivative with a detectable label. Examples of detectable labels include luminescent, enzymatic, and radioactive label. Both the type of label and the position of the label can effect analog activity. Labels should be selected and positioned so as not to substantially after the activity of the ghrelin analog at the GHS receptor. The effect of a particular label and position on ghrelin activity can be determined using assays measuring ghrelin activity and/or binding.

A protecting group covalently joined to the C-terminal carboxy group reduces the reactivity of the carboxy terminus under in vivo conditions. The carboxy terminus protecting group is preferably attached to the a-carbonyl group of the last amino acid. Preferred carboxy terminus protecting groups include amide, methylamide, and ethylamide protecting group is preferably attached to the a-carbonyl group of the last amino acid. Preferred carboxy terminus protecting groups include amide, methylamide, and ethylamide.

Suitable peptidyl analogues include those which are disclosed in US 2005/0148515 and which are incorporated herein by reference.

The present invention features ghrelin peptidyl and non-peptidyl analogs active at the GHS receptor.

Certain amino adds present in compounds of the invention are represented herein as follows:
A3c 1-amino-1cyclopropanecarboxylic acid
A4c 1-amino-1- cyclobutanecarboxylic acid
A5c 1-amino-1- cyclopentanecarboxylic acid
A6c 1-amino-1- cyclohexanecarboxylic acid
Abu α- aminobutyric acid
Acc 1-amino-1-cyclo(C₃ C₉) carboxylic acid
Act 4-amino-4-carboxytetrahydropyran, i.e.,: 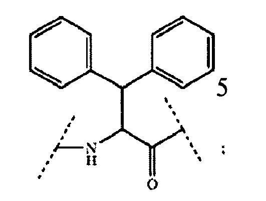
Aib α-aminoisobutyric acid
Ala or A alanine
β-Ala beta-alanine
Apc amino piperidinylcarboxylic aid, i.e.: 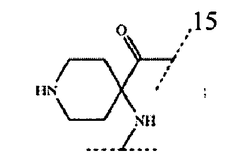
Arg or R arginine
hArg homoarginine
Asn or N asparagine
Asp or D aspartic acid
Bal 3-Benzothienylalanine, i.e.: 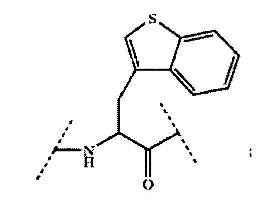
Bip 4,4'-Biphenylalanine, i.e.: 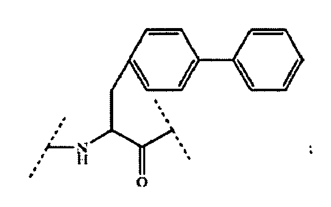
4-Benzoylphenylalanine, i.e.: 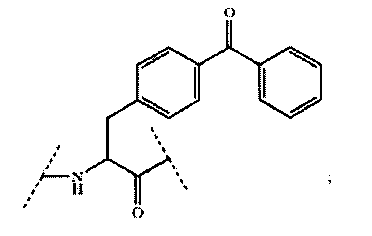
Cha β-cyclohexyalanine;
Cys or C cysteine;
Dab 2,4-diaminobutyric acid, (α,γ-Diaminobutyric acid);
Dap 2,3-diaminopropionic acid, (α,β-Diaminopropionic add)
Dip β,β-Diphenylalanine, i.e.: 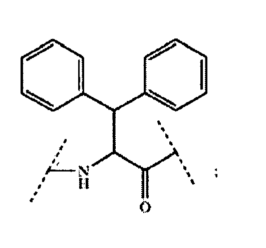
Dhp 3,4-dehydroproline
Dmt 5,5-dimethylthiazolidine-4-carboxylic acid
2Fua β-(2-furyl)-alanine, i.e.: 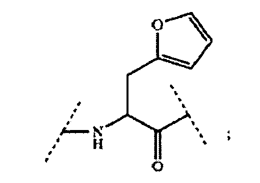
Gin or Q glutamine
Glu or E glutamic acid
Gly or G glycine
His or H histidine
3Hyp trans-3-hydroxy-L-proline, i.e., (2S, 3S)-3-hydroxypyrrolidine-2-carboxylic acid;
4Hyp 4-hydroxyproline, i.e., (2S, 4R)-4-hydroxypyrrolidine-2-carboxylic acid;
Ile or I isoleucine
Inc indoline -2-carboxylic acid
Inp isonipecotic acid, i.e.: 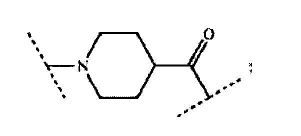
Ktp 4-ketoproline
Leu or L leucine
hLeu homoleucine
Lys or K lysine
Met or M methionine
1 Nal β-(1-Naphthyl)alanine;
2Nal β-(2-Naphthyl)alanine;
Nle norleucine
Nva norvaline
Oic octahydroindole-2-carboxylic acid
Om ornithine
2Pa1 ß-(2-Pyridyl)-alanine, i.e., 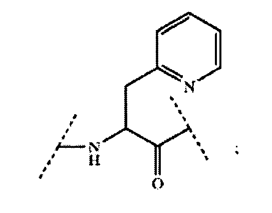
3Pal β-(3-Pyridyl)-alanine, i.e.: 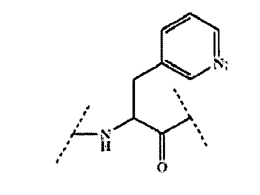
4Pal ß-(4-Pyridyl)-alanine, i.e.: 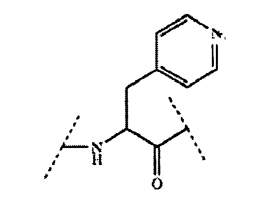
Pff pentafluorophenylalanine, i.e. 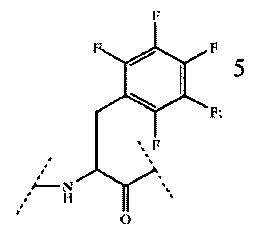
Phe or F phenylalanine
hPhe homophenylalanine
Pim 2'-(4-Phenyl)imidazolyl, i.e.: 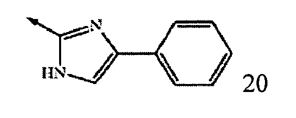
Pip pipecolic acid
Pro or P proline
Ser or S serine
Taz β-(4-thiazolyl)alanine, i.e., 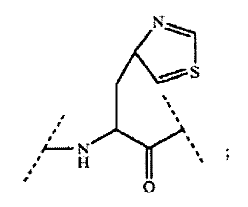
2Thi β-(2-thienyl)alanine, i.e.: 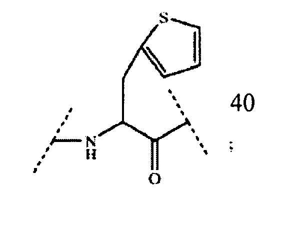
3Thi β-(3-thienyl)alanine, i.e.: 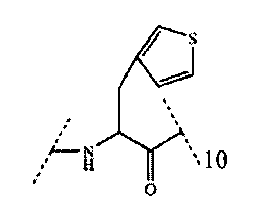
Thr or T threonine
Thz thiazolidine-4-carboxylic acid
Tic 1,2,3,4-tetrahydroisoquinoline-carboxylic acid
Tle tert-leucine
Trp or W tryptophan
Tyr or Y tyrosine
Val or V valine

Certain other abbreviations used herein are defined as follows:
Boc: tert-butyloxycarbonyl
Bzl: benzyl
DCM: dichloromethane
DIC: N,N-diisopropylcarbodiimide
DIEA: diisopropylethyl amine
Dmab:4{N-(1-(4,4-dimethyl-2,6-dioxocyclohexylidene)-3-methylbutyl)-amino}benzyl
DMAP:4-(dimethylamino)pyridine
DMF dimethylformamide
DNP:2,4-dinitrophenyl
Fmoc: Fluorenylmethyloxycarbonyl
HBTU:2-(1H-benzotriazole-1-yl)-1,1,3,3-tetramethyluronium hexafluorophosphate
cHex cyclohexyl
HOAT:O-(7-azabenzotriazol-1-yl)1,1,3,3-tetramethyluronium hexafluorophosphate
HOBt 1-hydroxy-benzotriazole
HOSu: N-hydroxysuccinimide
Mmt 4-methoxytrityl
NMP: N-methylpyrrolidone
Pbf: 2,2,4,6,7-pentamethyldihydrobenzofuran-5-sulfonyl
tBu: tert-butyl
TIS: triisopropylsilane
TOS: tosyl
trt trityl
TFA: trifluoro acetic acid
TFFH: tetramethylftuoroforamidinium hexafluorophosphate
Z: benzyloxycarbonyl

Unless otherwise apparent, abbreviations (e.g. Ala) of amino acids in this disclosure stand for the structure of -NH-C(R)(R')-CO-, wherein R and R' each is, independently, hydrogen or the side chain of an amino acid (e.g., R=CH₃ and R'=H for Ala), or R and R' may be joined to form a ring system.

"Alkyl" refers to a hydrocarbon group containing one or more carbon atoms, where multiple carbon atoms if present are joined by single bonds. The alkyl hydrocarbon group may be straight-chain or contain one or more branches or cyclic groups.

"Substituted alkyl" refers to an alkyl wherein one or more hydrogen atoms of the hydrocarbon group are replaced with one or more substituents selected from the group consisting of halogen, (i.e., fluorine, chlorine, bromine, and iodine), -OH, -CN, -SH, -NH₂, -NHCH₃, -NO₂, -C₁₋₂ alkyl substituted with 1 to 6 halogens, CF₃, -OCH₃, -OCF₃, and - CH₂)₀₋₄-COOH. In different embodiments 1, 2, 3 or 4 substituents are present. The presence of -(CH₂)₀₋₄-COOH results in the production of an alkyl acid. Examples of alkyl acids containing, or consisting of, -CH₂)₀₋₄ COOH include 2-norbornane acetic acid, tert-butyric acid and 3-cyclopentyl propionic acid.

"Heteroalkyl" refers to an alkyl wherein one of more of the carbon atoms in the hydrocarbon group are replaced with one or more of the following groups: amino, amido, -0-, or carbonyl. In different embodiments 1 or Z heteroatoms are present.

"Substituted heteroalkyl" refers to a heteroalkyl wherein one or more hydrogen atoms of the hydrocarbon group are replaced with one or more substituents selected from the group consisting of halogen, (i.e., fluorine, chlorine, bromine, and iodine), -H, -CN, -SH, -NH₂, - NHCH₃, -NO₂, -C₁₋₂ alkyl substituted with 1 to 6 halogens, -CF₃, -OCH₃, -OCF₃, and - CH₂)₀₋₄ COOH. In different embodiments 1, 2, 3 or 4 substituents are present.

"Alkenyl" refers to a hydrocarbon group made up of two or more carbons where one or more carbon-carbon double bonds are present. The alkenyl hydrocarbon group may be straight-chain or contain one or more branches or cyclic groups.

"Substituted alkenyl" refers to an alkenyl wherein one or more hydrogens are replaced with one or more substituents selected from the group consisting of halogen (i.e., fluorine, chlorine, bromine, and iodine), -H, -CN, -SH, ⁻NH₂, -NHCH₃, -NO₂, -C₁₋₂ alkyl substituted with 1 to 6 halogens, -CF₃, -OCH₃, OCF₃, and -(CH₂)₀₋₄COOH. In different embodiments 1, 2, 3 or 4 substituents are present.

"Aryl" refers to an optionally substituted aromatic group with at least one ring having a conjugated pi-electron system, containing up to two conjugated or fused ring systems. Aryl includes carbocyclic aryl, heterocyclic aryl and biaryl groups. Preferably, the aryl Is a 5 or 6 membered ring. Preferred atoms for a heterocyclic aryl are one or more sulfur, oxygen, and/or nitrogen. Examples of aryl include phenyl, 1-naphthyl, 2-naphthyl, indole, quinoline, 2-imidazole, and 9-anthracene. Aryl substituents are selected from the group consisting of -C₁₋₄ alkyl, -C₁₋₄ alkoxy, halogen (i.e., fluorine, chlorine, bromine, and iodine), -OH, -CN, OH, NH₂, -NO₂, -C₁₋₂ alkyl substituted with 1 to 5 halogens, -CF₃, - OCF₃, and - (CH₂)₀₋₄ COOH. In different embodiments the aryl contains 0, 1, 2, 3, or 4 substituents.

"Alkylaryl" refers to an "alkyl" joined to an "aryl".

When a non-amino acid imidazole moiety, (e.g., Pim, defined above), is present at the C-terminus of a compound of the invention It Is understood that the imidazole moiety Is attached to the adjacent amino acid via a pseudo-peptide bond, wherein a bond is formed between the position 2 carbon of the imidazole ring and the alpha carbon of the amino acid. For example, in the case where the adjacent amino acid is D-tryptophan (D-Trp) and the imidazole moiety is Pim, the C-terminus of the peptide would appear as follows:

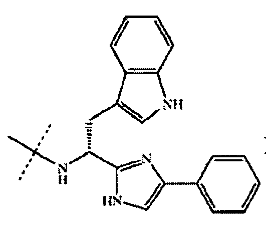

For clarity, in the written formula for such a compound the presence of this bond is indicated by the Greek letter "ψ" alone in parentheses. For example, the written formula H-Inp-D-Trp-D-2Nal(ψ)-Pim denotes the structure:

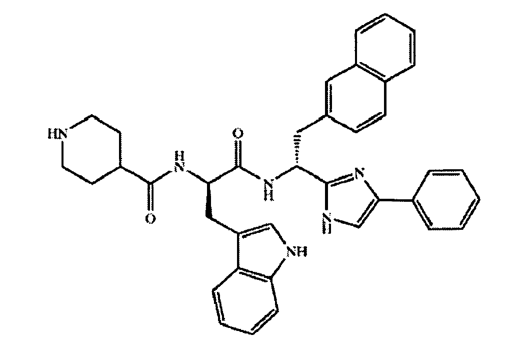

The compounds of the invention can be formulated and administered to a subject using the guidance provided herein along with techniques well known in the art. The preferred route of administration ensures that an effective amount of compound reaches the target Guidelines for pharmaceutical administration in general are provided in, for example, Remington's Pharmaceutical Sciences 18th Edition, Ed. Gennaro, Mack Publishing, 1990, and Modern Pharmaceutics 2nd Edition, Eds. Banker and Rhodes, Marcel Dekker, Inc., 1990, both of which are hereby incorporated by reference herein.

The compounds of the invention can be prepared as acidic or basic salts. Pharmaceutically acceptable salts (in the form of water- or oil-soluble or dispersible products) include conventional non-toxic salts or the quaternary ammonium salts that are formed, e.g., from inorganic or organic acids or bases. Examples of such salts include acid addition salts such as acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartate, thiocyanate, tosylate, and undecanoate; and base salts such as ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine and lysine.

Ghrelin and its analogues may be further modified e.g. may be acylated or unacylated. Such peptides are described e.g. in US 2005/0080007 and are incorporated herein by reference.

Homologues of ghrelin are also included into this invention. The term "Homology" refers to sequence similarity between two peptides while retaining an equivalent biological activity. Homology can be determined by comparing each position in the aligned sequences. A degree of homology between amino acid sequences is a function of the number of identical or matching amino acids at positions shared by the sequences so that an homologues sequence refers to a sequence sharing homology and an equivalent function or biological activity. The activity which should be retained by all peptides according to the present invention is to mimic ghrelin action on the GHS-receptor and other putative pathways. They further stimulate hair growth according to the persent invention. Due to the high sequence homology between ghrelins of different species (only 2 aa exchanged between rat and human ghrelin (Kojima et al., Nature 402:656-660 (1999)), all natural variants of ghrelin with more than 80 % identity, preferably more than 90 %, more preferably more than 95 %, and even more preferably more than 99 % with human ghrelin are encompassed as homologues of ghrelin by the present invention.

General methods and synthetic strategies used in providing functional and structural analogues of peptides is described in publications such as "Solid phase peptide sythesis" by Stewart and Young, W.H. Freeman & Co., San Francisco, 1969 and Erickson and Merrifield, "The Proteins", Vol. 2, p 255 et seq. (Ed. Neurath and Hill) Academic Press, New York, 1976.

The agent selected from the group comprising ghrelin, an analogue thereof and a pharmaceutically acceptable salt thereof may be administered through a route selected from the group consisting of intravenous, subcutaneous, transdermal, or oral (only non-peptide analoga)

Active ingredients to be administered orally as a suspension can be prepared according to techniques well known in the art of pharmaceutical formulation and may contain microcrystalline cellulose for imparting bulk, alginic acid or sodium alginate as a suspending agent, methylcellulose as a viscosity enhancer, and sweeteners/flavoring agents. As immediate release tablets, these compositions may contain microcrystalline cellulose, dicalcium phosphate, starch, magnesium stearate and lactose and/or other excipients, binders, extenders, disintegrants, diluents and lubricants.

The compounds of the invention may also be administered in intravenous (both bolus and infusion), intraperitoneal, subcutaneous, topical with or without occlusion, or intramuscular form. When administered by injection, the injectable solution or suspension may be formulated using suitable non-toxic, parenterally-acceptable diluents or solvents, such as Ringer's solution or isotonic sodium chloride solution, or suitable dispersing or wetting and suspending agents, such as sterile, bland, fixed oils, including synthetic mono- or diglycerides, and fatty acids, including oleic acid.

In a preferred embodiment the agent is selected from a group consisting of amino acid sequences according SEQ ID No. 1 -5.
1) AAU93610 (human)
   SEQ ID No. 1: GSSFLSPEHQRVQQRKESKK PPAKLQPR the third serine residue S is a n-Octanoyl modified serine
2) AAU93611 (rat)
   SEQ ID No.2: GSSFLSPEHQKAQRKESKKP PAKLQPR the third serine residue S is a n-Octanoyl modified serine
3) NP 067463 (mouse)
   SEQ ID No.3: GSSFLSPEHQKAQQRKESKKPPAKLQPR
4) BAC75929 (dog)
   SEQ ID No.4: GSSFLSPEHQKLQQRKESKKPPAKLQPR
5) BAD34670
   SEQ ID No.5: GSSFLSPEHQKVQQRKESKKPPAKLQPR

In another preferred embodiment the agent is administerd by an implanted osmotic pump in a therapeutically effective amount.

In another preferred embodiment the agent is topically administered in a therapeutically effective amount.

Suitable dosing regimens are preferably determined taking into account factors well known in the art including type of subject being dosed; age, weight, sex and medical condition of the subject the route of administration; the renal and hepatic function of the subject; desired effect; and the particular compound employed.

Optimal precision in achieving concentrations of drug within the range that yields efficacy without toxicity requires a regimen based on the kinetics of the drug's availability to target sites. This involves a consideration of the distribution, equilibrium , and elimination of a drug.

Further embodiment of this invention is a method for stimulating hair growth of a mammal comprising a therapeutically effective amount of an agent selected from the group comprising ghrelin, an analogue thereof and a pharmaceutically acceptable salt thereof.

In a preferred embodiment of this method the agent is selected from the group consisting of amino acid sequences according to SEQ ID No. 1 -5.

The agent selected from the group comprising ghrelin, an analogue thereof and a pharmaceutically acceptable salt thereof may be administered through a route selected from the group consisting of intravenous, subcutaneous, transdermal, or oral (only non-peptide analoga).

The agent selected from the group comprising ghrelin, an analogue thereof and a pharmaceutically acceptable salt thereof may be administered through a route selected from the group consisting of intravenous, subcutaneous, transdermal, or oral (only non-peptide analoga)

Active ingredients to be administered orally as a suspension can be prepared according to techniques well known in the art of pharmaceutical formulation and may contain microcrystalline cellulose for imparting bulk, alginic acid or sodium alginate as a suspending agent, methylcellulose as a viscosity enhancer, and sweeteners/flavoring agents. As immediate release tablets, these compositions may contain microcrystalline cellulose, dicalcium phosphate, starch, magnesium stearate and lactose and/or other excipients, binders, extenders, disintegrants, diluents and lubricants.

The compounds of the invention may also be administered in intravenous (both bolus and infusion), intraperitoneal, subcutaneous, topical with or without occlusion, or intramuscular form. When administered by injection, the injectable solution or suspension may be formulated using suitable non-toxic, parenterally-acceptable diluents or solvents, such as Ringer's solution or isotonic sodium chloride solution, or suitable dispersing or wetting and suspending agents, such as sterile, bland, fixed oils, including synthetic mono- or diglycerides, and fatty acids, including oleic acid.

In another preferred embodiment the agent is administered by an implanted osmotic pump in a therapeutically effective amount.

In another preferred embodiment the agent is topically administered in a therapeutically effective amount.

The therapeutically effective amount of the agent is between 10 nM/kg/d and 1000 nM/kg/d, preferred between 50 nM/kg/d and 700 nM/kg/d, more preferred between 50 nM/kg/d and 500 nM/kg/d, more preferred between 200 nM/kg/d and 500 nM/kg/d, with 500 nM/kg/d being the more effective dose.

In case of the administration by an implanted osmotic pump the pharmaceutical effective composition may be 2% inactivated serum and 5% Tween 80 in 0.9% saline. This might be understood as one example but is not meant as limitation.

The agents and methods may be used to support and stimulate the natural hair growth. Another application of the present invention is the regeneration of hair after operation or after hair loss due to chemotherapy or stress-related hair loss or hormone related hair loss.

The patent and scientific literature referred to herein represents knowledge that is available to those with skill in the art. All patents, patent publications and other publications cited herein are hereby incorporated by reference in their entirety.

It is to be understood that while the invention has been described in conjunction with the detailed description thereof, that the foregoing description is intended to illustrate and not limit the scope of the invention, which is defined by the scope of the appended claims. Other aspects, advantages, and modifications are within the claims.

### Figure Description

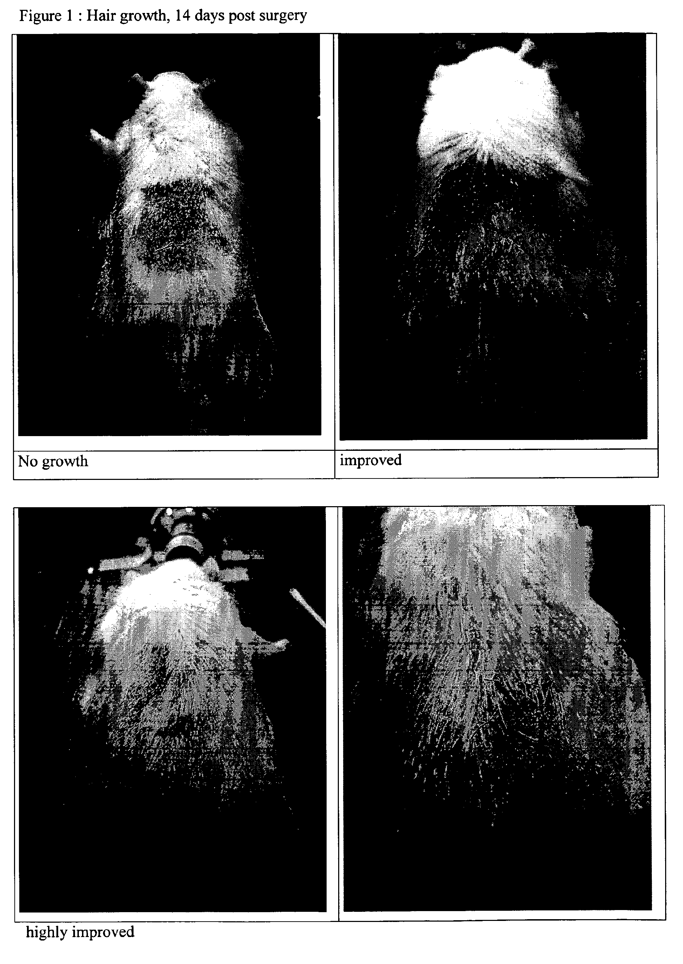

Figure 1 shows hair growth of rats in shaved areas, where the osmotic pumps were inserted, 14 days post surgery. Depending on the dose given the differences in hair growth were seen over a period of 14 days.

### Examples

### Methods

Left coronary artery ligation was performed on Sprague Dawley (SD) rats to induce a myocardial infarction (body weight at the time of surgery approx. 220 g). Alternatively, rats received sham-surgery. Twenty-six days after surgery, the animals were randomized to 8 infarct and 2 sham groups (sham: 12 animals per group; infarct: 18 animals per group at the start of therapy) and received one of three ghrelin-analoga or placebo via osmotic pumps implanted under the skin of the back.

### Results

### Macroscopical Pathology

In the shaved areas, where the osmotic pumps were inserted, differences in hair growth were seen over a period of 14 days. Images of the areas were acquired and scored in a blinded fashion.

| | | | |
|---|---|---|---|
| ghrelin, 50nM | n=9 | ghrelin, 500nM | n=10 |
| ghrelin analogue 1, 50nM | n=8 | ghrelin analogue 1, 500nM | n=10 |
| ghrelin analogue 2, 50nM | n=8 | ghrelin analogue 2, 500nM | n=10 |
| Sham, Placebo | n=4 | Sham, Plac., diuretic | n=10 |

| | Hair growth | | | |
|---|---|---|---|---|
| | no growth | normal | improved | highly improved |
| BIM 28125, 50nM | 4 | 3 | 1 | |
| BIM 28131, 50nM | | 2 | 6 | |
| BIM 28142, 50nM | 6 | 3 | | |
| BIM 28125, 500nM | 6 | 3 | 1 | |
| BIM 28131, 500nM | 6 | 2 | 2 | |
| BIM 28142, 500nM | | | 3 | 7 |
| Sham, Placebo | 2 | 2 | | |
| Sham, Plac.,Diuret. | 5 | 5 | | |
| | | | | |

The scores were analysed by the Chi-square test, resulting in X²= 76.63 und p < 0.001.

## Claims

1. Use of an agent selected from the group comprising ghrelin, an analogue thereof and a pharmaceutically acceptable salt thereof in the preparation of a medicament for administering to a mammal so as to stimulate its hair growth.

2. Use of an agent according to claim 1 whereas that agent is selected from a group consisting of amino acid sequences according SEQ ID No. 1 - 5.

3. Use of an agent according to any of claims 1 or 2 whereas the agent is administered by an implanted osmotic pump in a therapeutically effective amount.

4. Use of an agent according to any of claims 1 or 2 whereas the agent is administered topically in a therapeutically effective amount.

5. Method for stimulating hair growth of a mammal comprising a therapeutically effective amount of an agent selected from the group comprising ghrelin, an analogue thereof and a pharmaceutically acceptable salt thereof.

6. Method for stimulating hair growth of a mammal according to claim 5 comprising a therapeutically effective amount of an agent selected from the group consisting of amino acid sequences according SEQ ID No. 1-5.

7. Method for stimulating hair growth of a mammal according to claim 5 or 6 whereas the agent is administered by an implanted osmotic pump in a therapeutically effective amount.

8. Method for stimulating hair growth of a mammal according to claim 5 or 6 whereas the agent is topically administered in a therapeutically effective amount.
